# EUROPEAN PATENT APPLICATION

(11) **EP 3 505 614 A1**
(43) Date of publication of application: **03.07.2019**
(21) Application number: 17837223.1
(22) Date of filing: 01.08.2017
(51) Int. Cl.: C12M 3/00, B29C 67/00, C12N 5/077, C12N 5/071, B33Y 70/00

(54) **CELL CULTURE OBTAINED USING INTEGRATED THREE-DIMENSIONAL CELL PRINTING TECHNIQUE, AND METHOD FOR PREPARING SAME**

(30) Priority: 01.08.2016 KR 20160098109
(71) Applicant: T&R Biofab Co. Ltd., Siheung-si, Gyeonggi-do 15073 (KR)
(72) Inventor: CHO, Dong-Woo, Seoul 05065 (KR); KIM, Byoung Soo, Pohang-si Gyeongsangbuk-do 37673 (KR)
(74) Representative: Hoppe, Georg Johannes
(86) International application number: PCT/KR2017/008301
(87) International publication number: WO 2018/026172

(57) **Abstract**

The present invention relates to a method for preparing a dermis layer-like structure by a three-dimensional cell printing technology, a cell culture structure prepared by the method, for example, a method for manufacturing a dermis layer-like structure or artificial skin, and a cell culture structure prepared by the method, for example, a dermis layer-like structure or artificial skin, and the artificial skin manufactured by the three-dimensional printing manufacturing method according to the present invention is useful for mass-production and can be used as a base technology for development of artificial skin model further closer to actual skin.

## Description

### [TECHNICAL FIELD]

The present invention relates to a cell culture structure comprising a culture scaffold, and a cell layer prepared by 3-dimensional cell printing technology, and a method for preparing thereof.

### [BACKGROUND ART]

Artificial skin has been developed not only for wound healing but also for development of new drugs/cosmetics and skin basic research. In particular, as animal experiments for development of cosmetics are completely banned, the development of artificial skin is becoming more and more urgent. However, currently used artificial skin models for animal replacement are manufactured only by manual three-dimensional cell culture, and thus have the following limitations.

First, there is a difficulty in repetitive production and mass-production. Second, the artificial skin that has been successfully manufactured by this is sold at a very high price. Third, there are limitations in developing a skin model which considers various factors of real skin (pigment, blood vessel, aging, etc.). Therefore, in order to develop artificial skin more similar to human skin, introduction of new technology is definitely necessary. The present research is to develop an integrated cell printing technology considering the structural/physiological characteristics of human skin and to use it for development of artificial skin.

For development of artificial skin models, conventional researches require the use of an expensive transwell. However, since commercialized transwells are standardized in size, it is difficult to manufacture various sizes of artificial skin models. In addition, because of the use of expensive product and the entire manufacturing process performed by a person, accordingly, artificial skin is sold at a considerably expensive price.

In recent years, as the three-dimensional printing technology capable of precisely positioning various cells at a desired position has been spotlighted, the possibility of overcoming the conventional limitations and manufacturing a three-dimensional printed artificial skin model like real skin has been raised. Nevertheless, there is a problem that the printed artificial skin should be eventually transferred onto a commercialized transwell, and this problem may damage the printed artificial skin having relatively poor mechanical properties. In addition, there is a disadvantage that the three-dimensional printed artificial skin should be transferred onto a standardized transwell, although it can be manufactured in various shapes and sizes, and thus the advantage of the three-dimensional cell printing technology is offset.

In addition, one of the biggest issues in development of artificial skin models is contraction during the culture period. This contraction is caused by properties of hydrogels with poor mechanical properties, when cells adhere to the extracellular matrix. The contraction causes scars in the in vivo environment and has a limitation in culturing artificial skin for a long time in vitro. In the conventional manufacturing process, to overcome this problem, by positioning hydrogels which do not encapsulate cells on hydrogels which encapsulate cells before it is manufactured, the contraction is inhibited. However, this consumes expensive hydrogels, and these manufacturing process has a limitation in terms of repeatability and cost of development of artificial skin.

### [DISCLOSURE]

### [TECHNICAL PROBLEM]

One example of the present invention is to provide a cell culture structure prepared by printing a culture scaffold using thermoplastic resin and a cell layer comprising a cell and a gelling polymer integrally by a three-dimensional printing method, for example, a cell culture structure which can prepare a dermis-like structure or artificial skin, for example, a method for preparation of a dermis-like structure or artificial skin.

Additional one example of the present invention is to provide a method for preparation of a cell culture structure, for example, a dermis-like structure or artificial skin, which can prepare a scaffold and a cell layer comprising a cell integrally by a three-dimensional printing method, thereby solving a problem that a printed dermis layer or artificial skin comprising a cell should be moved on a transwell to culture it and in this case, a damage on the printed dermis layer or artificial skin having relatively poor mechanical properties is caused, and a limitation of applying various shapes and sizes of artificial skin prepared by a three-dimensional printing method to a standardized transwell.

Another example of the present invention is to provide a cell culture structure, for example, a dermis layer-like structure or artificial skin, in which a scaffold and a cell layer comprising a cell are integrally prepared by a three-dimensional printing method.

### [TECHNICAL SOLUTION]

The present invention can prepare a cell culture structure, for example, a dermis-like structure or artificial skin, by preparing an outer partitioning-wall member of a scaffold and a cell layer by a three-dimensional printing method of an extrusion type module using a synthetic biomaterials, and printing bioink comprising a gelling polymer solution and a cell on upper part of the scaffold and inside of the outer partitioning-wall member by a three-dimensional printing method, considering structural/physiological properties of skin and necessary conditions for development of artificial skin.

The specific method of printing the bioink by a three-dimensional printing method can be conducted by using both an extrusion type of module capable of spraying a high concentration of solution and a spray-type of module capable of spraying a low concentration of solution in small quantity.

According to the present invention, a two-layer structure of dermis layer-like structure comprising a scaffold and a dermis layer or a three-layer structure of artificial skin comprising a scaffold, a dermis layer and an epidermis layer can be integrally prepared by a three-dimensional printing method, thereby solving a problem that a printed dermis layer or artificial skin comprising a cell should be moved on a transwell to culture it and in this case, a damage on the printed dermis layer or artificial skin having relatively poor mechanical properties is caused, and a limitation of applying various shapes and sizes of artificial skin prepared by a three-dimensional printing method to a standardized transwell.

The present invention relates to an integrated cell printing technology, since hydrogels, which encapsulate a dermal fibroblast, prepared by the conventional methods are contracted during a culture period or storage period, the contraction of hydrogels can be inhibited by developing a dermis layer-like structure comprising a scaffold by a three-dimensional printing method.

Hereinafter, the present invention will be described in more detail.

One example of the present invention relates to a culture scaffold comprising a lower support member prepared with thermoplastic resin and an outer partition member located along the upper outside of the lower support member. The culture scaffold according to the present invention itself does not print a cell, and performs a function as a scaffold for culture of a cell layer located on its upper part, and has a porous structure comprising pores for smooth passage of culture solution.

Specifically, it relates to a culture scaffold comprising a porous lower support member which comprises two or more of print layers containing at least two or more of print lines located at an interval by an extrusion type of three-dimensional printing method, and pores which are formed by the print lines of different print layers located to have a certain crossing angle, and an outer partitioning-wall member formed along the upper outside of the lower support member.

Another example of the present invention relates to an integrated cell culture structure comprising a culture scaffold comprising a lower support member prepared with thermoplastic resin and an outer partitioning-wall member located along the upper outside of the lower support member; and a cell layer prepared on upper part of the lower support part and inside of the outer partitioning-wall member of the culture scaffold by a three-dimensional printing method, and the cell layer may be a two-layer structure comprising a dermis layer, or a three-layer structure comprising a dermis layer and an epidermis layer.

Additional one example of the present invention relates to a cell layer isolated from an integrated culture structure comprising the culture scaffold and the cell layer, and it may be a one-layer structure comprising a dermis layer, or a one-layer structure comprising a dermis layer and an epidermis layer.

Other one example of the present invention relates to a dermis layer-like structure comprising a culture scaffold comprising a lower support member prepared with thermoplastic resin and an outer partitioning-wall member located along the upper outside of the lower support member; and a similar dermis layer which is laminated on upper part of the lower support member and comprises a gelling polymer and a fibroblast.

In addition, one example of the present invention relates to artificial skin which is laminated on upper part of the dermis layer-like structure and comprises a similar epidermis layer comprising a gelling polymer and an epidermal cell.

The lower support member has a porous structure having pores, and a dermis layer and an epidermis layer are located on upper part of the lower support member and inside of the outer partitioning-wall member and both the lower support member and the cell layer are prepared by a series of three-dimensional printing method, and thus the shape, size and pores of the lower support member can be appropriately controlled to be suitable for a desired cell layer. Modifications such as contraction of the dermis layer can be prevented, since the movement of culture solution is smooth during cell culture and the dermis layer and/or the epidermis layer can be physically supported and the size of the porous structure can be controlled, due to pores inside of the lower support member, and the size of the porous structure of the lower support member can be controlled, thereby helping the first layer of the dermis layer to be fused well with the lower support member.

The pores of the culture scaffold mean internal space formed during printing the first print layer comprising two or more of print lines (internal partition wall, fiber), and the second print layer comprising two or more of print lines crossed to form a certain crossing angle with the print lines comprised in the first print layer. The crossing angel may be prepared to various angles, and when the crossing angle is 90°, the lattice pattern is formed, and the print layer may be prepared as two layers or more. In specific one example, it may be a culture scaffold comprising; the plural first print lines which are extended along the first direction and are arranged at a certain interval along the second direction which is crossed with the first direction; and the plural second print lines which are extended along the second direction on the plural first print lines and are arranged at a certain interval along the first direction, wherein the plural first print lines and the plural second print lines relatively comprise a thermoplastic resin.

The culture scaffold according to the present invention comprises a lower support member and an outer partitioning-wall member located along the upper outside of the lower support member, and the lower support member comprises two layers or more of print layers containing at least two or more of print lines located at an interval, and the print lines of the adjacent print layers may comprises pores formed by printing to have a certain crossing angle, and it may be laminated in multiple layers to prepare a scaffold of desired height. In one example of the present invention, the culture scaffold equipped with both the lower support member and the outer partitioning-wall member has an effect of significantly reducing contraction of the dermis layer formed on its top, compared to the scaffold which comprises only the outer partitioning-wall member or comprises only the internal print line. According to such an effect of prevention of contraction, it means that not only it is possible to culture the dermis layer and the epidermis layer in vitro for a long time more stably, but also it has an effect of inhibiting scars when applied in vivo for wound healing.

The thickness or width of the print lines may be 0.1 to 0.5mm, 0.1 to 0.4mm, 0.1 to 0.3mm, or 0.1 to 0.2m, but not limited thereto. The printing height of the print lines may be 0.1 to 1.0 mm, 0.1 to 0.9 mm, 0.1 to 0.8 mm, 0.1 to 0.7 mm, 0.1 to 0.6 mm, 0.1 to 0.5 mm, 0.1 to 0.4 mm, or 0.1 to 0.3 mm, for example, 0.2 mm. The size of the pores formed on the lower support member may be 100 to 1000 um, 100 to 900 um, 100 to 800 um, 100 to 700 um, 100 to 600 um, 100 to 500 um, 100 to 400 um, 100 to 300 um, for example, 200 um, but not limited thereto. Since the culture scaffold according to the present invention is prepared by a three-dimensional printing method, pores correspond to the distance or interval between the adjacent print lines in the same print layer. The distance between the adjacent print lines in the same print layer may include 100 to 1000 um.

The lower support member of the culture scaffold may have pores, and the size of pores, that is the distance between the adjacent print lines in the same print layer may be same or different. When the distance of the adjacent print lines in the same print layer is different, the interval of the adjacent print lines of the upper same print layer may be 300 to 1,000 micrometers and the interval of the adjacent print lines of the lower same print layer may be 100 to 500 micrometers. By diversifying the size of pores of the lower support member, the lower layer part consists of relatively big size of pores (200µm∼1000µm) capable of smooth supply of culture solution, and the upper layer part is where the dermis layer is printed and relatively small size of pores are arranged, and therefore, the function of preventing contraction of the dermis layer can be performed.

The outer partitioning-wall member may be same as the height of the cell layer comprising a cell or higher than it, and for example, when the thickness of the cell layer (length-micrometer basis) is 1, the height of the outer partitioning-wall member may be 1 to 5. The outer partitioning-wall member has an effect of preventing contraction of the cell layer formed on upper part of the lower support member, and thus it means that it has effects capable of not only culturing the cell layer in vitro for a long time stably, but also inhibiting scars when applied in vivo for wound healing.

The culture scaffold may use thermoplastic resin as material, and for example, it may be one or more kinds selected from the group consisting of polycaprolactone (PCL), poly(lactate-co-glycolate) (PLGA), poly lactic acid (PLA), polyurethane (PU), poly(lactide-co-caprolactone (PLCL), polydioxanone (PDO), polystyrene (PS), and preferably, it may be polycaprolactone (PCL). Since polycaprolactone is sprayed at a relatively low temperature, it has an advantage of having high cell viability when printed with a cell.

The culture scaffold may be prepared by filling a gelling polymer in pores formed in the print lines, by using a temperature-sensitive gelling polymer as a sacrificial layer, and the sacrificial layer may be removed from the culture scaffold.

The dermis-like structure according to the present invention comprises a culture scaffold comprising the lower support member and the outer partitioning-wall member located along the upper outside of the lower support member, and a dermis layer comprising bioink including a fibroblast and a gelling polymer, which is prepared by printing with an extrusion type of three-dimensional printing method.

The dermis layer-like structure is prepared by an integrated process applying a three-dimensional printing method to the culture scaffold and the dermis layer, and thereby the bioink is laminated on the upper layer of the lower support member, and thus the binding capacity of the scaffold and the similar dermis layer is strong. Since the cross-linking binding of the gelling polymer comprised in the bioink is performed in the laminated condition, there is an effect of significantly reducing contraction of the dermis layer, compared to the case of attaching the dermis layer obtained by performing cell layer printing and cross-linking separately without the culture scaffold to a separate culture scaffold, for example, a transwell, according to the conventional methods.

In addition, in the dermis-like structure according to the present invention, the dermis layer may (i) contain a fibroblast in the entire cell layer, or (ii) form cell containing region and non-containing region in the vertical direction by not comprising a cell in the lower dermis layer adjacent to the lower support member and comprising a cell only in the upper dermis layer, or (iii) form cell containing region and non-containing region in the horizontal direction by comprising a cell in its inside by being spaced by a certain interval with the outer partitioning-wall member of the culture scaffold, for example, 50 micrometers or more, for example, 50 to 5000 micrometers distance or 100 to 1000 micrometers distance, or (iv) form cell containing region and non-containing region in both the vertical direction and the horizontal direction. The cell containing region may perform a three-dimensional printing by using bioink containing a cell, and the cell non-containing region may prepare perform a three-dimensional printing by using bioink not containing a cell, in order to prepare a dermis layer. As the spaced distance of the outer partitioning-wall member and the cell layer, the distance suitable for preventing contraction of the cell layer may be set to a lower limit value, and an upper limit value may be appropriately set considering the entire size of the cell layer.

Thus, the similar dermis layer comprised in the dermis layer-like structure may has a cross-sectional reduction ratio of 25% or less, 24% or less, 23% or less, 22% or less, 21% or less, 20% or less, 19% or less, 18% or less, 17% or less, 16% or less, 15% or less, 14% or less, 13% or less, 12% or less, or 11% or less, based on the initial dermis layer cross-sectional area of 100, after cell culture. This means that it has effects of not only making possible to culture in vitro for a long time more stably but also inhibiting scars when applied in vivo for wound healing.

The similar dermis layer may be sprayed so that the height is 0.1 to 3.5 mm, 0.1 to 3.0 mm, 0.1 to 2.5 mm, 0.1 to 2.0 mm, 0.1 to 1.5 mm, 0.5 to 3.5 mm, 0.5 to 3.0 mm, 0.5 to 2.5 mm, 0.5 to 2.0 mm, or 0.5 to 1.5 mm, for example, 1.0 mm, but not limited thereto.

One example of the present invention provides artificial skin which is laminated on upper part of the dermis layer-like structure, and comprises a similar epidermis layer comprising a gelling polymer and an epithelial cell. The epithelial cell may be prepared by culturing and differentiating artificial skin comprising a dermal keratinocyte.

It should have junction between the cell and cell, and should be located as a confluent mono-layer on the dermis layer before differentiation. The epithelial cell located on such a dermis layer is exposed in air and starts to be differentiated to form epidermis. Thus, to prepare artificial skin, a cell existed in the dermis layer should be surrounded by three-dimensional extracellular matrix, and the contraction of the dermis layer encapsulating the cell should be minimized, and the epithelial cell should express normal cell viability and protein and consist of a confluent mono-layer, and the artificial skin to be prepared should be possible to grow as mature skin by forming both submerge condition and air-liquid interface condition.

Since the artificial skin prepared by the conventional three-dimensional printing methods should prepare a dermis layer and an epidermis layer separately and move them to a culture scaffold, for example, a transwell, it diminishes the advantage of the cell printing technology which can manufacture it in various sizes and shapes, and also there is possibility to be damaged during the process of moving the dermis layer and epidermis layer which have poor mechanical properties right after printing to a transwell. The integrated cell printing system which integrally prepares a culture scaffold and a dermis layer, or a dermis layer and an epidermis layer by a three-dimensional printing method according to the present invention may satisfy the structural/physiological characteristics of skin, and therefore, it can solve all the existing problems of repeatability, mass-productivity, various shapes and sizes, complicated manufacturing process and expensive cost, and further, makes it possible to manufacture artificial skin like real skin.

Accordingly, all of the culture scaffold, dermis layer and epidermis layer can be prepared by the integrated three-dimensional printing method. Specifically, a porous culture scaffold consisting of a lower support member and an outer partitioning-wall member is prepared by using thermoplastic resin through an extrusion type module, and a similar dermis layer is prepared by printing bioink comprising a fibroblast and a gelling polymer on the lower support member by an extrusion type of three-dimensional printing method, and a dermis layer-like structure is prepared by performing cross-linking the gelling polymer. Next, an epidermis layer is prepared on the similar dermis layer with bioink comprising an epithelial cell and a gelling polymer by the three-dimensional printing method. Thus, the artificial skin prepared according to the present invention may manufacture artificial skin comprising (one step) air-liquid interface condition without the use of an expensive regulated transwell and additional work at a time.

In addition, in the artificial skin according to the present invention, when the dermis layer consists of a region containing a cell uniformly, or comprises cell containing region and cell non-containing region, the epidermis layer may prepare cell containing region as same as the upper region of the dermis layer. In other words, when the dermis layer comprises a cell in the entire regions, the epidermis layer may be formed on the entire upper part of the dermis layer, and when the dermis layer forms cell containing region and non-containing region in the horizontal direction by spacing at a certain interval from the outer partitioning-wall member of the culture scaffold, for example, at a distance of 50 micrometers or more, for example, 50 to 5000 micrometers or 100 to 1000 micrometers, and comprising a cell in its inside, the epidermis layer may be formed only in the upper part of the cell containing region.

The dermis-like structure or artificial skin according to the present invention is prepared by printing bioink containing a cell and a gelling polymer to a dermis layer and an epidermis layer by a three-dimensional printing method. The culture scaffold and dermis layer may be prepared by an extrusion type three-dimensional printing method using relatively high viscosity bioink, and the epidermis layer may be prepared by an extrusion type or inkjet type of three-dimensional printing method.

The viscosity of bioink comprising a cell and a gelling polymer may be adjusted to maintain suspension of the cell and also preferably, make it fluid enough for dispersion, for example, by injection through a needle or nozzle.

The bioink used in the present invention includes for example, a hydrogel mixing/non-mixing a desired cell, a hydrogel containing a growth factor, a hydrogel containing a cell and/a growth factor, a hydrogel containing a cytokine, different kinds of hydrogels, etc. The gelling polymer may be one or more kinds selected from the group consisting of collagen, gelatin, Matrigel, alginate, agarose, decellularized tissue-derived cell ink, hyaluronic acid and fibrin gel, and the hydrogel prepared by the gelling polymer has advantages of high moisture content, excellent biocompatibility, controllable mechanical properties and excellent biodegradability.

The gelling polymer according to the present invention is preferably a gelling polymer which can form a hydrogel, and more preferably, it may be a hydrogel-forming polymer having temperature-sensitivity, for example, collagen and gelatin. As collagen has bioaffinitive and temperature-sensitive properties, and gelatin shows temperature-sensitive properties, they are particularly suitable as the cell delivery material. In other words, gelatin has properties that is liquefied at 37°C and is solidified at a room temperature or below.

The bioink may further comprise a tissue-derived component to intensify tissue specificity, and for example, the tissue-derived component may be that specific tissues of animal such as cartilage, kidney, heart, liver, muscle, etc. is decellularized and a substance containing extracellular matrix as a main component is gelated. The bioink may further comprise a cell culture medium and the cell culture medium includes any medium suitable for a target cell.

The bioink according to the present invention may comprise a cell, and an applicable cell or tissue is not particularly limited, and it may be an animal cell or a plant cell, or an animal or plant tissue. The cell may be one or more selected from the group consisting of stem cell, osteoblast, myoblast, tenocyte, neuroblast, fibroblast, glioblast, germ cell, hepatocyte, renal cell, Sertoli cell, chondrocyte, epithelial cell, cardiovascular cell, keratinocyte, smooth muscle cell, cardiomyocyte, glial cell, endothelial cell, hormone-secreting cell, immunocyte, pancreatic islet cell and neuron. In case of preparing artificial skin, the dermis layer may comprise a fibroblast and the epidermis layer may comprise an epithelial cell.

The cell type used in the artificial tissue prepared of the present invention may be culture by any method known in the art. The cell and tissue culture methods are known in the art.

In the present invention, 'bioprinting' means to use three-dimensional accurate cell deposition (e.g. cell solution, cell containing gel, cell suspension, cell concentrate, multicellular aggregate, multicellular body, etc.) through automated, computer-based, three-dimensional prototyping devices (e.g. bioprinter) and commonly used methodology. The three-dimensional printing may be performed by extruding a biocompatible polymer from a nozzle using a bio-plotter, thereby laminating it on a stage.

The bioink can facilitate cross-linking of a bioink composition, by heating it, or being exposed to ultraviolet rays, or adding a cross-linking solution, after laminated by being sprayed by a three-dimensional bioprinter. Such cross-linking makes the laminated bioink composition completed as a harder structure. To facilitate the cross-linking, a photoinitiator may be used.

Other example of the present invention may prepare a dermis-like structure comprising a culture scaffold and a dermis layer integrally by using a three-dimensional printing method, or prepare artificial skin comprising a culture scaffold, a dermis layer and an epidermis layer.

A specific method for preparation of a dermis-like structure may comprise,
(a) a step of preparing a culture scaffold comprising an outer partitioning-wall member located along the upper outside of a lower support member,
(b) a step of preparing a similar dermis layer by printing bioink containing a fibroblast and a gelling polymer on the lower support member by an extrusion type of three-dimensional printing method, and
(c) a step of forming cross-linking of the gelling polymer.

A method for preparation of artificial skin according to the present invention may comprise,
(a) a step of preparing a culture scaffold comprising an outer partitioning-wall member located along the upper outside of a lower support member,
(b) a step of preparing a similar dermis layer by printing bioink containing a fibroblast and a gelling polymer on the lower support member by an extrusion type of three-dimensional printing method,
(c) a step of forming cross-linking of the gelling polymer,
(d) a step of preparing a similar epidermis layer by printing bioink containing a dermal keratinocyte and a gelling polymer on the similar dermis layer by a three-dimensional printing method, and
(e) a step of culturing and differentiating the keratinocyte.

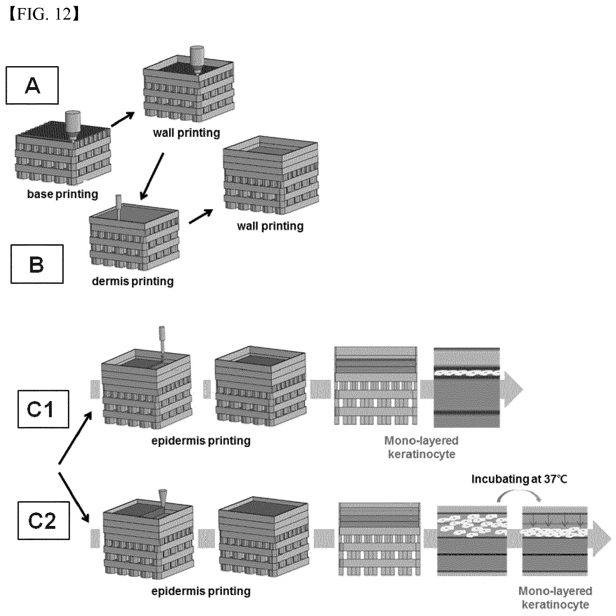

The dermis-like structure or artificial skin according to the present invention is prepared by printing bioink containing a cell and a gelling polymer on a dermis layer and an epidermis layer by a three-dimensional printing method. The culture scaffold and dermis layer may be prepared by an extrusion type of three-dimensional printing method using relatively high-viscosity of bioink, and the epidermis layer may be prepared by an extrusion type or inkjet type of three-dimensional printing method (See FIG. 12). The step of preparing artificial skin according to the present invention may integrally prepare a two-layer structure of dermis layer-like structure comprising a culture scaffold and a dermis layer by a three-dimensional printing method, or a three-layer structure of artificial skin comprising a scaffold, a dermis layer and an epidermis layer by a three-dimensional printing method.

According to one example of the present invention, a culture scaffold, a dermis layer and an epidermis layer may be integrally printed by applying a three-dimensional printing method, and the order of printing of the culture scaffold, the dermis layer and the epidermis layer may be in order of printing a lower support member and an outer partitioning-wall member of the culture scaffold primarily, printing the dermis layer, printing an outer partitioning-wall member secondarily, and printing the epidermis layer, or, in order of sequentially printing the dermis layer and the epidermis layer, after printing a lower support member and an outer partitioning-wall member of the culture scaffold.

For example, one example of preparation of artificial skin of the present invention is shown in FIG. 12, and specifically, a culture scaffold consisting of a lower support member and an outer partitioning-wall member may be prepared by an extrusion type of three-dimensional printing method, and a dermis layer may be prepared on upper part of the lower support member and inside of the outer partitioning-wall member by an extrusion type of three-dimensional printing method, and an outer partitioning-wall member may be prepared along the outside of the dermis layer by an extrusion type of three-dimensional printing method, and then an epidermis layer may be prepared inside of the prepared outer partitioning-wall member by an inkjet type of three-dimensional printing method using low-viscosity of bioink or an extrusion type of three-dimensional printing using high-viscosity of bioink.

In addition, in order to further prevent contraction of a cell layer, the dermis-like structure according to the present invention may print cell containing region and cell non-containing region in the vertical direction or the horizontal direction, and for example, (i) in addition to containing a fibroblast in the entire cell layer, (ii) may form cell containing region and cell non-containing region in the vertical direction by comprising a cell only in the upper dermis layer without comprising a cell in the lower dermis layer, or (iii) may form cell containing region and cell non-containing region in the horizontal direction by comprising a cell in its inside by being spaced at a certain interval with the outer partitioning-wall member of the culture scaffold, for example, 50 micrometers or more, for example, 50 to 5000 micrometers, or 100 to 1000 micrometers in distance, or (iv) may form cell containing region and cell non-containing region in both the vertical direction and horizontal direction by combining the (ii) and (iii).

The cell containing region may perform three-dimensional printing by using bioink containing a cell and the cell non-containing region may perform three-dimensional printing by using bioink non-containing a cell, to prepare a dermis layer.

The cell, bioink and gelling polymer are same as described above in the dermis-like structure and artificial skin. Hereinafter, the method for preparation of the dermis-like structure and artificial skin according to the present invention will be described in detail by each step.

### (a) The step of preparing a culture scaffold comprising the lower support member and the outer partitioning-wall member located along the upper outside of the lower support member

The culture scaffold according to the present invention may comprise a lower support member and an outer partitioning-wall member located along the upper outside of the support member, and the lower support member may comprise two layers or more of print layers containing at least 2 or more of print lines located at an interval by an extrusion type of three-dimensional printing method, and the print lines of the different print layers may have a porous structure comprising pores formed by printing to have a certain crossing angle, and it may be laminated by two layers or more.

Specifically, the culture scaffold, may prepare a print layer comprising two or more of print lines by spraying thermoplastic resin and spraying the thermoplastic resin on a plate for printing, by an extrusion type of three-dimensional printing method. The step of manufacturing the culture scaffold comprising two or more of print lines may further perform a step of preparing a sacrificial layer by spraying and filling gelatin to formed pores, and preparing a print layer comprising 2 or more of print lines by spraying thermoplastic resin on the scaffold in which gelatin is filled, thereby laminating print layers once or more, and it may be carried out to prepare a scaffold having a desired height. The thermoplastic resin, printing width and height of print lines (internal partition wall, fiber), and formation of the crossing angle of print lines comprised in different print layers are same as described above. The height of the lower support member may be 500 to 3,000 micrometers, and the height of the outer partitioning-wall member may be 1,000 to 5,000 micrometers.

The thermoplastic resin may be sprayed as fused by heating it for 10 min to 20 min to be 70°C to 90°C, after encapsulating it in a syringe. The concentration of the gelatin for preparation of the sacrificial layer may be 20 to 45 % by weight, 20 to 40 % by weight, or 20 to 35 % by weight. When the concentration of the gelatin is less than 20 % by weight, since the gelatin becomes solution before the printed collagen is cross-linked and thus it cannot play a role of the sacrificial layer, the use in the above range makes collagen play a role of the sacrificial layer for cross-linking enough and also makes later removal easy.

### (b) The step of preparing a similar dermis layer by printing bioink containing a fibroblast and a gelling polymer on the lower support member by an extrusion type of three-dimensional printing method

This is the step of printing a dermis layer by an extrusion type of three-dimensional printing method by using bioink comprising a fibroblast and a gelling polymer on the lower support member, after preparing a culture scaffold comprising a lower support member and an outer partitioning-wall member located along the upper outside of the support member by the three-dimensional printing method according to the present invention, and may (i) contain a fibroblast in the entire cell layer, or (ii) form cell containing region and non-containing region in the vertical direction by comprising a cell only in the upper dermis layer without comprising a cell in the lower dermis layer, or (iii) form cell containing region and non-containing region in the horizontal direction by comprising a cell in its inside by being space at an interval with the outer partitioning-wall member of the culture scaffold, or (iv) form cell containing region and non-containing region in both the vertical direction and the horizontal direction by combining the (ii) and (iii).

The cell containing region may perform three-dimensional printing by using bioink containing a cell, and the cell non-containing region may perform three-dimensional printing by using bioink non-containing a cell, to prepare a dermis layer. The cell, gelling polymer, bioink and height of the dermis layer are same as described above.

### (c) The step of forming cross-linking of the gelling polymer

The step of preparing a culture scaffold comprising a lower support member and an outer partitioning-wall member, printing a dermis layer containing a fibroblast and a gelling polymer on the top, and forming cross-linking of the gelling polymer, according to the extrusion type of three-dimensional printing method of the present invention.

The cross-linking of a bioink composition may be facilitated by heating it, exposing it to ultraviolet rays or adding a cross-linking solution. Such cross-linking makes the laminated bioink composition completed as a harder structure. To facilitate the cross-linking, a photoinitiator may be used.

When forming cross-linking by using collagen as a gelling polymer for preparation of the dermis layer and heating it, since cross-linking of collagen is not occurred at a temperature less than 37°C, it may be performed at a temperature of 37°C or more, and for example, it may be performed at 37 to 40°C, 37 to 39°C, or 37 to 38°C. In addition, the collagen bioink may have the concentration of collagen of 1 %(w/v) to 10 %(w/v), for example, 1 to 5%(w/v), 1.5 to 4.5%(w/v) or 1.5 to 3%(w/v) or, 1.5(w/v), but not limited thereto.

Specifically, the collagen bioink may be one in which human dermal fibroblasts are mixed to a solution in which 2%(w/v) collagen solution and fetal bovine serum are mixed at a volume ratio of 10:1 to 5:1, so that the density of cells is 2.5 x 10⁵ cells/ml to 1 x 10⁶ cells/ml.

The dermis layer-like structure prepared by the method for preparation have strong binding capacity of the similar dermis layer that the scaffold and collagen bioink form, as the print line on the uppermost layer of the lower support member and the collagen bioink are sequentially prepared and the collagen bioink is laminated in the space between print lines comprised in the same print layer, and the cross-linking of collagen is performed in this condition, and thus there is an effect of significantly lowering the initial reduction ratio of the cross-sectional area of the dermis layer, compared to the case of attaching collagen to the scaffold after cross-linking. Thus, in the similar dermis layer of the dermis layer-like structure, contraction is prevented even after cell culture, and this means that not only it is possible to culture the dermis layer and the epidermis layer in vitro for a long time more stably, but also it has an effect of inhibiting scars when applied in vivo for wound healing.

In case of using gelatin as a sacrificial layer when preparing the culture scaffold, the dermis-like structure is cultured in a cell incubator, and the gelatin of the sacrificial layer is washed out together with the cell medium, and finally, a porous scaffold manufactured with thermoplastic resin performs a role of a commercialized transwell.

### (d) The step of preparing a similar epidermis layer by printing bioink containing a dermal keratinocyte and a gelling polymer on the similar dermis layer by a three-dimensional printing method

The method for preparing artificial skin according to the present invention may comprise a step of preparing a similar epidermis layer by printing bioink containing a keratinocyte and a gelling polymer on the dermis-like structure by a three-dimensional printing method, and the epidermis layer may be prepared inside of the prepared outer partitioning-wall member by an inkjet type of three-dimensional printing method using low-viscosity of bioink or an extrusion type of three-dimensional printing method using high-viscosity of bioink, after preparing an outer partitioning-wall member along the outside of the dermis layer by an extrusion type of three-dimensional printing method.

When the epidermis layer is prepared by an inkjet type (or spray-type) of three-dimensional printing, relatively low viscosity of bioink may be used, and when it is printed by an extrusion type of three-dimensional printing method, relatively high viscosity of bioink may be used. Since a cell should form a confluent monolayer in the epidermis layer, when an inkjet type (or spray-type) of three-dimensional printing is performed by using cell-containing bioink, the confluent monolayer may be formed without performing an additional process, and when an extrusion type of three-dimensional printing is performed, after printing with bioink, a process of liquefying the gelling polymer at a certain temperature to prepare a monolayer cell layer may be carried out.

The inkjet type has an advantage which can locate a cell on a desired point with relatively high precision, and it is to solve that a non-uniform epidermis layer is prepared when progressed with the conventional pipette, and it enhances the function of forming epidermis and overcomes a problem of repeatability and mass-production that are the conventional limitations by exposing the epidermal cell in air after locating it on the dermis layer uniformly.

Since the extrusion type can prepare a three-dimensional structure by encapsulating a cell in a high viscosity of hydrogel and laminating it on a desired position, but it is difficult that the cell exist in a high concentration due to high thickness compared to the inkjet module, it can increase the cell concentration by modifying the gelling polymer comprised in bioink by temperature raising and sinking the cell down, and for this, it is preferable to use a thermosensitive gelling polymer, and for example, gelatin is advantageous, because it has characteristics of moving as solid at a low temperature (37°C or lower), but being changed to liquid (sol) at a high temperature (37°C or higher).

When the epidermis layer is prepared by using the extrusion type of three-dimensional printing method, after printing the epidermis layer with gelatin bioink comprising a cell by technology for printing a large amount of cells uniformly, the gelling polymer is changed to liquid in various conditions and internal cells are uniformly accumulated on the dermis layer. As the method for being changed to liquid, there is a method of using temperature change and salt concentration, etc., gelatin may be liquefied by temperature raising and collagen may be liquefied by temperature lowering.

In one example of the present invention, the bioink for forming an epidermis layer may have an appropriate viscosity range, and it may be achieved by adjusting the concentration of a gelling polymer. When using gelatin as the gelling polymer, since when the concentration of gelatin is high, the printing precision is improved but the strong property of the gelatin material is high, thereby causing a crack of the dermis layer, the content of gelatin comprised in bioink may be 1 %(w/v) to 10 %(w/v), for example, 1 to 5%(w/v), 1.5 to 4.5%(w/v) or 1.5 to 3%(w/v) or, 2.5%(w/v). There is no limitation on the concentration of the gelling polymer of the epidermis layer, for example, gelatin, particularly, but when the concentration is excessively high, the strength is increased, and thus damages of the dermis layer may be caused. When applying an inkjet type of three-dimensional printing method, there is no appropriate concentration, but when the concentration is high, it is generally preferable to use low viscosity of liquid, since inkjet extrusion is difficult.

### (e) The step of culturing a keratinocyte

The method for preparing artificial skin according to the present invention comprises a step of preparing an epidermis layer on a dermis-like structure and proliferating and differentiating a cell by performing culturing of the cell.

The cell culture step may be applied as same as the method used for common methods for preparation of artificial skin. For example, the culture step may be carried out at 35 to 40°C, 35 to 39°C, 35 to 38°C, 36 to 40°C, 36 to 39°C, 36 to 39°C, or 36 to 38°C, for example, 37°C. The culture step may be carried out for 12 to 36 hours, 12 to 32 hours, 12 to 28 hours, 16 to 36 hours, 16 to 32 hours, 16 to 28 hours, for example, 24 hours in a 5% CO₂ incubator.

The differentiation step may be carried out after removing gelatin by washing the dermis layer-like structure with phosphate buffered saline (PBS). The keratinocyte may be one that 6 x 10⁵ cell of keratinocytes are encapsulated in keratinocyte growth media and sprayed. It is divided into a proliferation step of increasing the number of cells by culturing cells and a differentiation step of expressing cell-specific properties, and since in case of skin tissue, the epidermal cell has a property to be differentiated when it is not sunk in a culture solution and is exposed in air, a process of exposing it in air (air-liquid interface, ALI) is required.

### [ADVANTAGEOUS EFFECTS]

The present invention relates to a method for preparing a dermis layer-like structure by a three-dimensional cell printing technology, a dermis layer-like structure prepared by the method, a method for manufacturing artificial skin using the dermis layer-like structure and artificial skin manufactured by the method, and the artificial skin manufactured by the three-dimensional printing manufacturing method according to the present invention is useful for mass-production and can be used as a base technology for development of artificial skin model further closer to actual skin.

### [DESCRIPTION OF DRAWINGS]

FIG. 1 is a mimetic diagram of three scaffolds to confirm the structure of the culture scaffold for overcoming contraction of the culture scaffold prepared by the three-dimensional printing method according to the example of the present invention.
FIG. 2 is real photographs taking collagen contraction of the dermis layer at day 1, day 3, day 5 and day 7 of the culture period during culturing the dermis-like structure prepared according to Example 1.
FIG. 3 shows a graph digitizing the contracted degree of collagen at day 1, day 3, day 5 and day 7 according to the example of the present invention.
FIG. 4 is photographs taking cellular morphology through live and dead assay of collagen bio-ink contracted for 7 days according to the example of the present invention by each group.
FIG. 5 is a process chart showing a one-step preparation process of artificial skin using an integrated cell printing technology according to the example of the present invention.
FIG. 6 are photographs showing rectangle shape and circle shape of artificial skin prepared using the integrated cell printing technology according to the example of the present invention.
FIG. 7 are photographs showing keratinocytes printed on collagen using the inkjet module according to the example of the present invention.
FIG. 8 is photographs showing keratinocytes located on collagen by a pipette as a comparison group to show possibility of the inkjet module according to the example of the present invention.
FIG. 9 is photographs showing the degree of formation of the epidermis layer of the artificial skin developed for 14 days, compared to the artificial skin prepared on the commercial transwell according to the example of the present invention.
FIG. 10 is graphs digitizing the thickness of the epidermis layer of the artificial skin at day 7 and day 14 according to the example of the present invention.

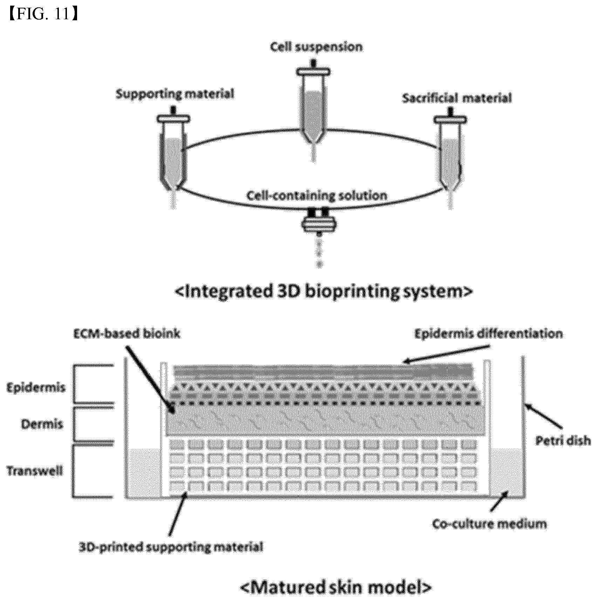

FIG. 11 is a drawing showing a cross-section of the artificial skin prepared according to the example of the present invention.
FIG. 12 is a schematic diagram showing a method for manufacturing a culture scaffold and artificial skin (including dermis and epidermis) according to the example of the present invention.
FIG. 13 shows the result of epidermal cell printing using gelatin (C2 in FIG. 12) according to the example of the present invention.
FIG. 14 shows the result of cell observation after epidermal cell printing using gelatin according to example of the present invention.
FIG. 15 is photographs showing the result of printing according to gelatin concentration according to the example of the present invention.
FIG. 16 is comparison of the result of the penetration of culture solution by the pore size of the lower support member of the culture scaffold according to example of the present invention.
FIG. 17 is a schematic diagram of description of contraction by cells of the artificial skin prepared according to the example of the present invention.
FIG. 18 is a schematic diagram showing the printing method of dermal and epidermis layers for preventing contraction by cells according to the example of the present invention.
FIG. 19 shows the result of contraction by the difference of the printing method of dermal and epidermis layers for preventing contraction by cells according to the example of the present invention.

### [MODE FOR INVENTION]

Hereinafter, the present invention will be described in more detail by the following examples. However, these examples are intended to illustrate the present invention only, and the scope of the present invention is not limited by these examples.

### Preparative example 1. Preparation of bioink (collagen bioink)

### (1) Collagen bioink

Collagen type 1 in a sponge form (Dalimtissen, Inc.) was dissolved in 0.02M acetic acid (DUKSAN) for a day at a room temperature (25°C) so that the concentration was 2%(w/v). Then, the acidic collagen was neutralized by using 0.5M NaOH (Sigma-Aldrich) immersed in ice to prepare approximately pH 7.0 of collagen solution.

Fetal bovine serum (FBS) was mixed to the neutralized collagen solution at a volume ratio of 10:1 (collagen solution:fetal bovine serum), and human dermal fibroblasts were mixed so that the density of cells were 2.5 x 10⁵ cells/ml, to prepare collagen bioink comprising collagen, fetal bovine serum and fibroblasts, and distilled water was added so that the concentration of the collagen in the collagen bioink was 1.5%(w/v), to prepare collagen bioink comprising collagen, fetal bovine serum and fibroblasts, and distilled water was added so that the concentration of the collagen in the collagen bioink was 1.5%(w/v), to prepare the final collagen bioink.

### (2) Gelatin bioink

After gelatin in a powder form was added to 1 X PBS by 10%(w/v), and was dissolved to the maximum at a room temperature, it was sterilized at the temperature of 120 °C for 20 min, or was prepared in a liquid state completely at the temperature of 65 °C or higher. The prepared gelatin solution was cooled at a room temperature and filtered through a 0.22 *µ*m filter in the liquid state.

The gelatin prepared for manufacturing an epidermis layer and dermal keratinocytes of 1×10⁷/ml or 5×10⁶/ml were mixed at a ratio of 4:6 and loaded in a printing syringe and stored at 4 °C or in ice to prepare it in a solid form.

### Example 1: Analysis of structure for preventing contraction of cell layer

### (1) Preparation of a similar dermis structure comprising a scaffold comprising PCL internal lattice and a dermis layer (A)

Polycaprolactone (hereinafter PCL) 1g was encapsulated in a syringe, and then was fused by heating it for 10 min to be 80°C. Then, the fused PCL was sprayed with a 600kPa of compressed dispenser (Musashi Engineering, Inc.) to prepare a scaffold in a PCL lattice pattern in the diameter of 12 mm and the height of 0.8 mm and prepare a scaffold comprising PCL internal lattice.

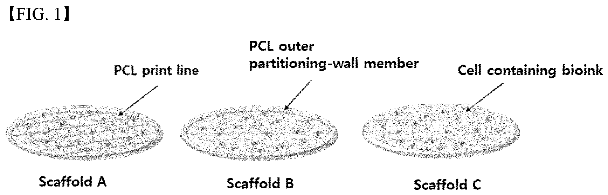

On the prepared scaffold, a similar dermis layer was printed by an extrusion type of three-dimensional printing method with the collagen bioink containing fibroblasts prepared in Preparative example 1 so that the height was 1 mm, and collagen was cross-linked at 37°C for 1 hour, to prepare a dermis layer-like structure having 13 mm of diameter and 2.0 mm of height (scaffold A). The mimetic diagram of the prepared dermis-like structure comprising the culture scaffold and dermis layer was shown in FIG. 1.

### (2) Preparation of a similar dermis structure comprising a scaffold having an outer partitioning-wall member and a dermis layer (B)

Polycaprolactone (hereinafter PCL) 1g was encapsulated in a syringe, and then was fused by heating it for 10 min to be 80°C. Then, the fused PCL was sprayed with a 600kPa of compressed dispenser (Musashi Engineering, Inc.) to prepare a PCL outer partitioning-wall member having 12 mm of diameter and 3.0 mm of height, and prepare a scaffold having the outer partitioning-wall member.

On the prepared scaffold, a similar dermis layer was printed by an extrusion type of three-dimensional printing method with the collagen bioink containing fibroblasts prepared in Preparative example 1 so that the height was 1 mm, and collagen was cross-linked at 37°C for 1 hour, to prepare a dermis layer-like structure having 13 mm of diameter and 2.0 mm of height (scaffold B). The mimetic diagram of the prepared dermis-like structure comprising the culture scaffold and dermis layer was shown in FIG. 1.

### (3) Preparation of a dermis-like structure comprising a dermis layer without a lower support member (C)

Without preparing the PCL partition wall, on a printing plate directly, a similar dermis layer was printed by an extrusion type of three-dimensional printing method with the collagen bioink containing fibroblasts prepared in Preparative example 1 so that the height was 1 mm, and collagen was cross-linked at 37°C for 1 hour, to prepare a dermis layer-like structure having 13 mm of diameter and 2.0 mm of height (scaffold C). The mimetic diagram of the prepared dermis-like structure comprising the culture scaffold and dermis layer was shown in FIG. 1.

### Experimental example 1. Measurement of degree of contraction of dermis layer

To investigate the degree of contraction of the dermis layer, the dermis-like structures prepared from scaffolds A, B, C of Example 1 were cultured in fibroblasts growth media (Dulbecco's Modified Eagle's medium with high glucose (DMEM/HG), 10% FBS, 1% Penicillin-Streptomycin) for 7 days by maintaining 37°C respectively and replacing media once in 2 days, and each dermis-like structure cultured for 7 days were washed using phosphate buffered saline (PBS).

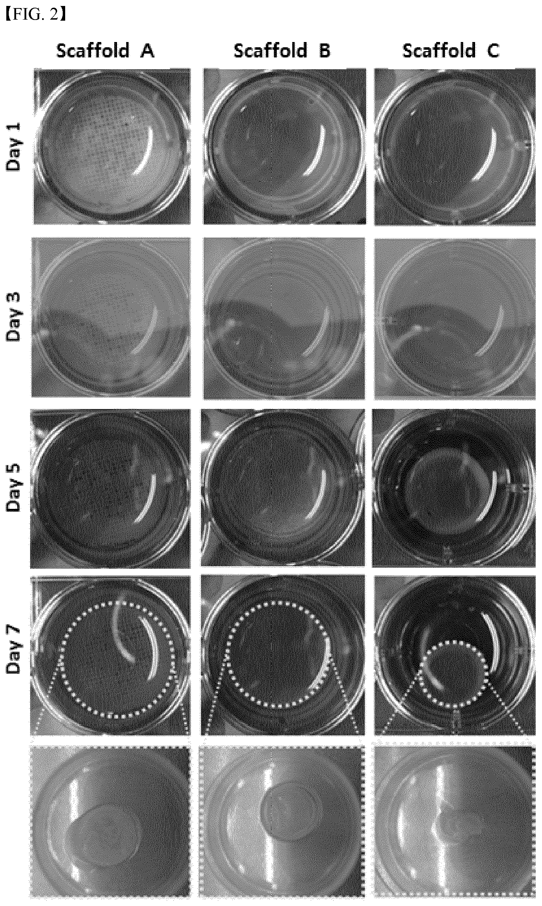

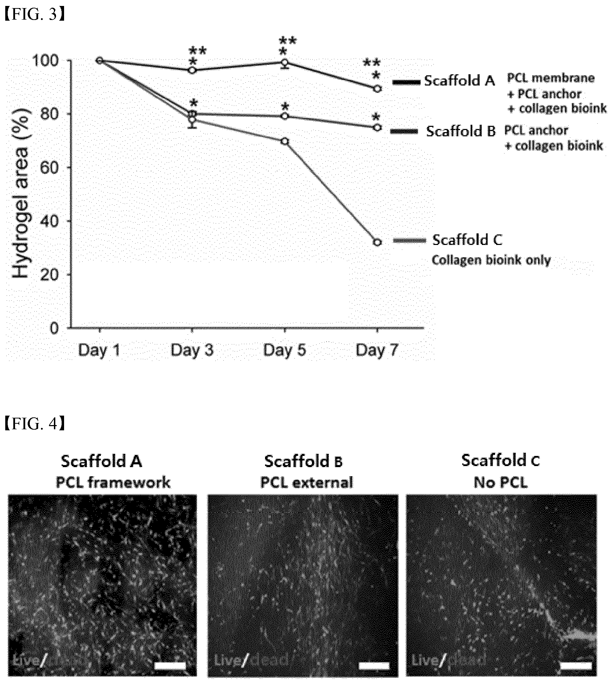

During culturing for 7 days, the degree of contraction of dermis layer structures was observed by using a microscope at day 1, day 3, day 5 and day 7, and the result was shown in FIG. 2, and to numerically measure the degree of contraction, the cross-section of contracted dermis layers was measured by using Image J software and represented as percentages and shown in FIG. 3 and Table 1. The following Table 1 means that the relative cross-sectional area ratio of the dermis layer based on the initial dermis layer cross-sectional area of 100 as the experimental date passes.

**[Table 1]**

| Percentage (%) | Day 1 | Day 3 | Day 5 | Day 7 |
|---|---|---|---|---|
| Scaffold A | 100 | 96.28838 | 99.30136 | 89.41122 |
| Scaffold B | 100 | 78.05246057 | 79.1797101 | 74.88301708 |
| Scaffold C | 100 | 97.57479015 | 68.2525173 | 32.46616975 |

As can be seen in FIG. 2, FIG. 3 and Table 1, it was confirmed that the reduction ratio of the cross-sectional area of the dermis layer should be 15% or less, but in case of scaffold A of Example 1, the cross-sectional area of the initially prepared structure was not reduced only by approximately 10% and thus the structure was maintained best. It was confirmed that the dermis layer-like structure of the present invention could overcome collagen contraction that was a limitation of the conventional dermis layer based on poor collagen which was prepared with a pipette (conventional). This means that when developing an artificial skin model, not only it makes it possible to culture in vitro for a long time more stably, but also it has an effect of inhibiting scars when applied in vivo for wound healing.

### Experimental example 2. Live and dead assay of fibroblasts

To investigate the effect of contraction of the dermis layer on a cell, after 7 days when contraction was completed, cell viability and cellular morphology were confirmed by using Live and dead assay.

Specifically, the dermis-like structures prepared from scaffolds A, B, C of Example 1 were cultured for 7 days by the same culturing method as Experimental example 1.

Then, to dye living cells to green and dead cells to red, a working solution was prepared by mixing 2uL EthD-4uM (Life Technologies) and 2uL calcein AM 2uM (Life Technologies) with 1mL of PBS buffer, respectively, and after immersing each structure to the prepared working solution for 20 min, images were photographed by using a fluorescent microscope, and the fluorescent microscope photographs were shown in FIG. 4.

As can be seen in FIG. 4, all the scaffolds A, B, C of Example 1 showed appropriate cell viability, but in the dermis-like structure in which contraction of scaffold A of Example 1 was inhibited, the most stable cellular morphology was observed.

### Example 2. Artificial skin manufacturing (A-B-C1 mode)

### (1) Culture scaffold preparation

By the same method as preparation in Example 1, on the plate for printing, a porous lower support member in a lattice pattern was printed with a PCL print line so as to have the height of 0.2 mm in a lattice pattern, and by printing the material of a sacrificial layer, 25% gelatin (Sigma-Aldrich) between the lattice of the lower support member so that there was no empty space, to prepare a scaffold in the thickness of 1mm. By performing three-dimensional printing along the upper outside of the prepared scaffold, a PCL outer partitioning-wall member was prepared so as to have the height of 0.2 mm, to prepared a culture scaffold having a lower support member equipped with an internal partition wall having pores in a lattice pattern and an outer partitioning-wall member.

### (2) Dermis layer preparation

On upper part of the lower support member and inside of the outer partitioning-wall member of the prepared culture scaffold, the collagen bioink containing fibroblasts prepared in Preparative example 1 by an extrusion type of three-dimensional printing method and collagen was cross-linked at 37°C for 1 hour, to prepare a dermis layer-like structure having 13 mm of diameter and 2.0 mm of height.

### (3) Epidermis layer preparation

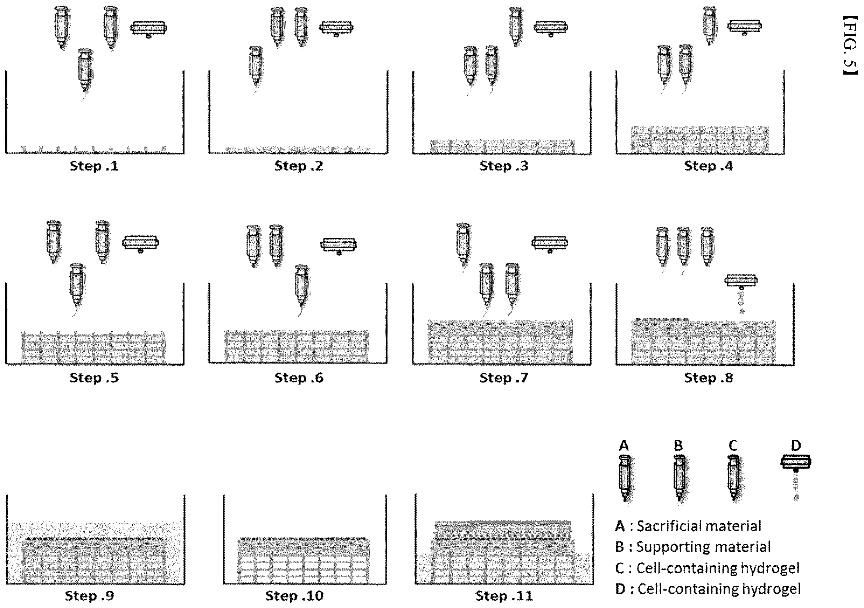

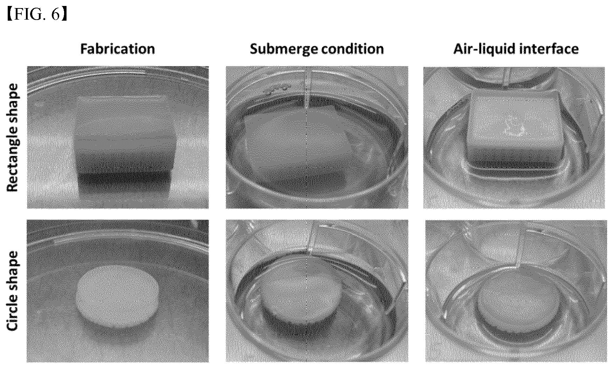

The mimetic diagram of preparation of the artificial skin was shown in FIG. 5, and photographs of the prepared circle shape structures and rectangle shape structures were shown in FIG. 6.

Specifically, on the dermis layer-like structure, the gelatin bioink in which epidermal cells of 6 x 10⁵ cell prepared in Preparative example 1 were encapsulated in 1 mL of keratinocyte growth media (Gibco™) was uniformly sprayed by a three-dimensional printing method.

Then, the structure in which the gelatin bioink comprising keratinocytes on the dermis layer-like structure was put in a culture container, and a fibroblast culture solution was filled until the dermis layer-like structure was sunk and it was put in a 37°C, 5% CO₂ incubator and put for a day. Since the gelatin sacrificial layer was solidified at a room temperature and liquefied at 37°C due to its properties, after culturing for a day, the material of the sacrificial layer was removed by PBS washing. Then, it was maintained as completely sunk in the culture solution for 3 to 5 days and its surface was exposed in air by reducing the volume of the culture solution for differentiation of keratinocytes, to prepare artificial skin.

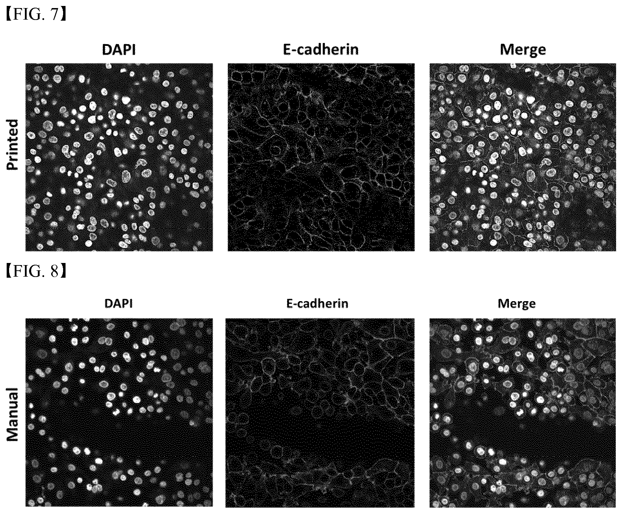

In FIG. 6, the method for preparation of artificial skin according to the present invention can prepare artificial skin having various shapes and sizes, and specifically, shows real images of printing a porous PCL culture scaffold which can play a role of a transwell with a dermis layer and an epidermis layer. In addition, in FIG. 8, there are photographs showing three circumstances which are necessary for development of artificial skin prepared by bioprinting (fabrication, submerge condition, air-liquid interface). The technology of preparation of artificial skin according to the present invention makes it possible to simplify the manufacturing process and manufacture low price of artificial skin in various shapes and forms.

### Comparative example 1

Artificial skin was prepared by the substantially same method as Example 2, but instead of performing three-dimensional printing the gelatin bioink comprising keratinocytes by the inkjet type in Example 2, artificial skin comprising a cell layer in which an epidermal cell was located on a dermis-like structure with a pipette was prepared.

### Experimental example 3. Keratinocyte distribution analysis (keratinocyte morphology)

The experiment was carried out in order to confirm whether an epidermal cell, keratinocyte was uniformly located on a dermis layer without encapsulation in a high concentration of hydrogel, when an epidermis layer was prepared by an inkjet type of three-dimensional printing method by using an integrated cell printing system according to the present invention.

Specifically, the artificial skin comprising the epidermis layer prepared by an inkjet type of three-dimensional printing method on a dermis layer-like structure according to Example 2, and the artificial skin in which a keratinocyte was sprayed on a dermis layer-like structure with a pipette according to Comparative example 1 were put in keratinocyte growth media for a day, and then nuclei of cells and cell to cell adhesion were observed through immune-staining (E-cadherin/DAPI), and the result was shown in FIG. 7 and FIG. 8.

As can be seen in FIG. 7 and FIG. 8, it was confirmed that when it was uniformly sprayed on the dermis layer-like structure by using the inkjet module according to Example 2, it was uniformly located in most of area (FIG. 7), but when the keratinocyte was located on the dermis layer-like structure by using a pipette according to Comparative example 1, the keratinocyte was ununiformly distributed, for example, there was empty space sparsely, etc., and in particular, the epidermal cell was hardly observed on the outermost side of the collagen hydrogel (FIG. 8).

In order that a keratinocyte is exposed in air to be normally differentiated, a confluent mono-layer should be formed when located on a dermis layer, but as the amount of bioink cannot be minutely adjusted in case of using a pipette, it takes time for the keratinocyte to escape from the dermis layer or to adhere to the dermis layer. This problem acts as a bad factor for formation of a confluent layer, and consequently it has limitations on repeatability and mass production of development of artificial skin.

On the other hand, the present invention can adjust bioink comprising a cell minutely, and thus keratinocytes could be uniformly filled on the dermis layer. This result may be used as one of key technologies which can overcome limitations such as complicated manufacturing process, low repeatability and difficulty of mass production, etc. that are conventional limitations, during development of artificial skin.

### Experimental example 4. Analysis of degree of formation of epidermis layer (epidermis)

An experiment to confirm whether the artificial skin prepared in Example 2 properly performs the alternative role of a real commercialized transwell (Corning Inc.) was progressed.

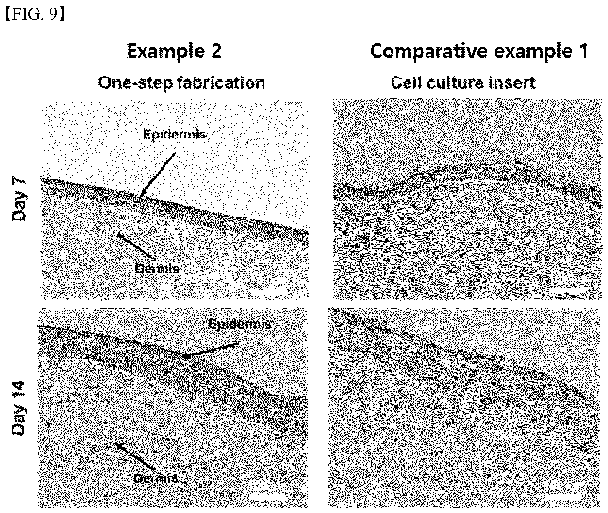

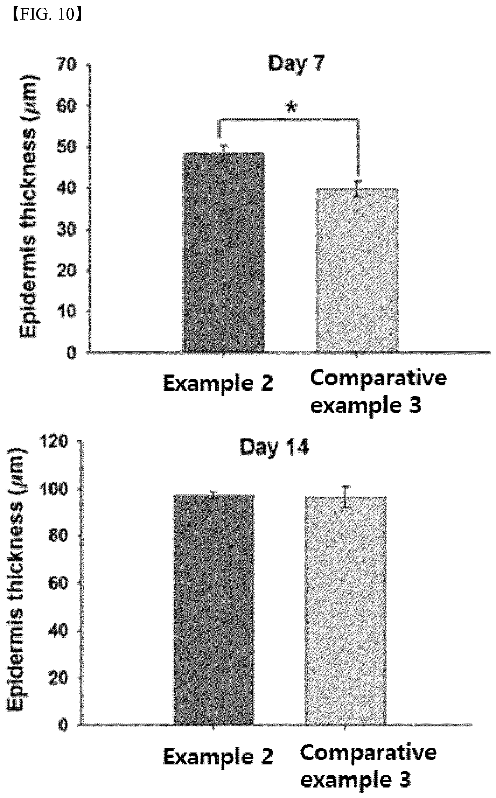

Specifically, after exposing the artificial skin prepared in Example 2 in air, Hematoxylin and Eosin staining was performed at day 7 and day 14, and the result was shown in FIG. 9. In addition, the thickness of the epidermis layer between each group was quantitatively measured by using Image J software and shown in FIG. 10 and Table 2. As a control group, the same method was performed by using the real commercialized transwell (Corning Inc.).

**[Table 2]**

| Thickness of epidermis layer (um) | Day 7 | Day 14 |
|---|---|---|
| Artificial skin of Example 2 | 48.50 | 97.25 |
| Artificial skin of control group | 39.75 | 96.25 |

As shown in FIG. 9, it was confirmed that the artificial skin of Example 2 represented in the left (one-step fabrication) was formed well without great significant difference in thickness of the epidermis layer, compared to Comparative example 1 using a transwell represented in the right (cell culture insert), and thereby it was confirmed that the cell porous scaffold according to the present invention could substitute the conventional expensive commercialized transwell. As shown in FIG. 10, it was confirmed that the thickness of the epidermis layer in the artificial skin of Example 2 was thicker than Comparative example 1 at day 7 and significant difference was not shown at day 14, and thereby it was confirmed that artificial skin could be manufactured with a dermis layer-like structure. The thickness of the epidermis layer means that the epidermis layer was differentiated well, and it was confirmed that the culture scaffold of the present invention can smoothly perform differentiation of the epidermis layer as same as the conventional commercialized transwell, by performing a function of providing an air-liquid interface condition. It could be seen that in Example 2, both the dermis layer and epidermis layer had uniform and dense arrangement of cells, and this means that it has a similar structural form with the real skin further more than the control group.

### Example 3: Manufacturing of artificial skin using gelatin dermis layer (A-B-C2 mode)

### (1) Preparation of gelatin bioink containing dermal keratinocytes

As same as Preparative example 1, after gelatin in a powder form was added to 1 X PBS by 10%(w/v), and was dissolved to the maximum at a room temperature, it was sterilized at the temperature of 120 °C for 20 min, or was prepared in a liquid state completely at the temperature of 65 °C or higher. The prepared gelatin solution was cooled at a room temperature and filtered through a 0.22 *µ*m filter in the liquid state. The gelatin prepared for manufacturing an epidermis layer and dermal keratinocytes of 1×10⁷/ml or 5×10⁶/ml were mixed at a ratio of 4:6 and loaded in a printing syringe and stored at 4 °C or in ice to prepare it in a solid form.

The gelatin bioink containing dermal keratinocytes was put in a syringe for printing and installed to a printing head set at 15∼25 °C in advance, and then it was kept for about 10∼30 min and stored until the viscosity became printable.

### (2) Preparation of dermis-like structure

As shown in FIG. 12, artificial skin in which all the scaffold, dermis layer and epidermis layer were prepared by an extrusion type of three-dimensional printing method was prepared. PCL (Polycaprolacton, Polyscience Inc.) was put in a steel syringe for polymers and PCL was dissolved for 10∼20 min by contracting it to a syringe heater set at 90∼140 °C (TCD-200EX), and then completed fused PCL was extruded with a 300 *µ*m nozzle and a lower support member and an outer partitioning-wall member were prepared by a three-dimensional printing method to prepare a culture scaffold (A).

For dermis layer (B) manufacturing, bioink in which a collagen-containing hydrogel and dermal fibroblasts were mixed was loaded in a syringe, the first layer was printed in an approximately 200 *µ*m thickness to be fused well in the upper part of the PCL support layer, and the second and third layers were printed in an approximately 800∼1,000 *µ*m of layer thickness, by an extrusion type of three-dimensional printing method under the printing conditions of nozzle 250 *µ*m, pneumatic 30∼100 kPa, transfer speed approximately 200mm/min.

### (3) Manufacturing of artificial skin

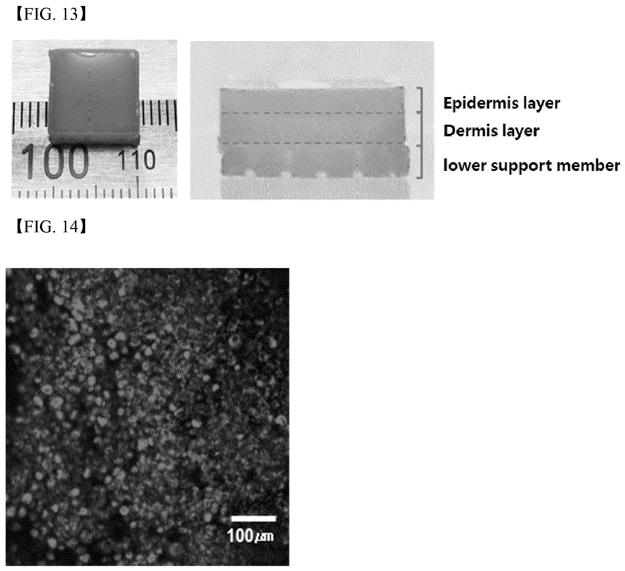

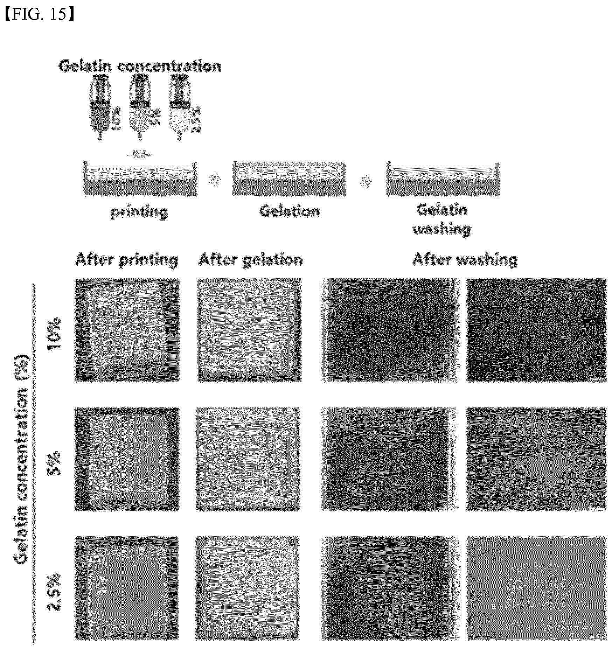

According to the mimetic diagram of artificial skin manufacturing of FIG. 15, to print epidermal cells in a thin monolayer structure, the epidermis layer (C2) was printed by using the gelatin bioink containing dermal keratinocytes. Specifically, the gelatin bioink at a concentration of 2.5 %(W/V) comprising epidermal cells of dermal keratinocytes 2 x 10⁶ cell was printed on the dermis-like structure under the conditions of nozzle 250∼400 *µ*m, pneumatic 15∼30 kPa and transfer speed 200∼500 mm/min by an extrusion type of three-dimensional printing method. The printed gelatin bioink was changed to liquid in an incubator (approximately 37 °C) and thereby the epidermal cells formed a monolayer on the dermis layer. The photograph of the obtained three-dimensionally printed artificial skin was shown in FIG. 13.

It was confirmed by a confocal microscope analysis method by attaching DiI which could exhibit cells as red to the epidermal cells comprised in the prepared artificial skin, and as FIG. 14, it was confirmed that a great quantity of cells were uniformly printed in high density.

### Example 4: Preparation of artificial skin using various concentrations of gelatin

Gelatin bioink was prepared by the substantially same method with the method for preparation of the gelatin bioink containing dermal keratinocytes according to Example 3, but bioink was prepared at three gelatin concentrations of 2.5%(w/v), 5%(w/v) and 10%(w/v). Using the prepared gelatin bioink, artificial skin was prepared by the substantially same method with Example 3.

The printing tendency according to the gelatin concentration was analyzed, and its photograph was shown in FIG. 15. In FIG. 15, the photograph right after printing (the leftmost column), the photograph right after forming cross-linking (the middle column) and the photograph after washing (the rightmost column) were shown. As the result of experiment for artificial skin comprising the epidermis layer prepared with the bioink using three gelatin concentrations, it was confirmed that when the concentration of gelatin was high, the printing precision was improved, but the strong property of the gelatin material was high, and therefore, a crack of the dermis layer was caused, and through this, the investigation of proper concentration (connected with the strong property) was progressed. Referring to the experimental result of FIG. 15, 2.5% gelatin was uniformly printed without causing a crack of the dermis layer, but on the other hand, at the gelatin concentration of 5% and 10%, a crack of the dermis layer was observed.

### Example 5: Manufacturing of artificial skin

Since a phenomenon that a culture scaffold was floated in a culture solution, when a culture scaffold (pore 300 *µ*m), in which the pore size of the lower support member (interval between print lines) was variously set, was injected to a cell culture solution, and thus, to solve this, an experiment of adjusting the pore size of the scaffold was progressed.

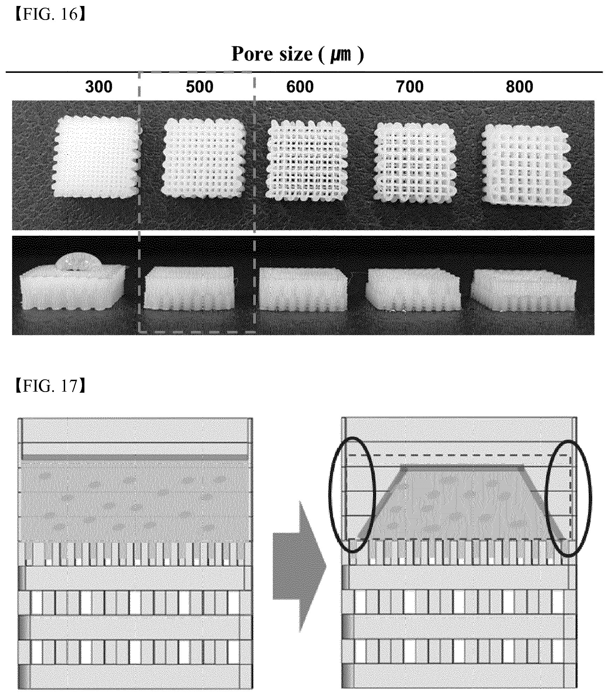

A lower support member of a culture scaffold was prepared by the substantially same method as Example 1, but a culture scaffold having various pores was manufactured, in which the pore size of the upper part of the scaffold was fixed to 300 *µ*m to prevent that dermis layer bioink escaped between pores, and the pore size of the lower part of the scaffold was fixed to 300∼800 *µ*m, specifically 300, 500, 600, 700 and 800 *µ*m for smooth circulation of the cell culture solution. Specifically, the scaffold was composed of 2 to 10 layers, and it was prepared as the uppermost layer had the pore size of 300 *µ*m, and the lower layer had pores as 300, 500, 600, 700 and 800 *µ*m uniformly. The photograph of the prepared culture scaffold was shown on top of FIG. 16. The pores of the lower support member correspond to the interval of printing regions when printing by a three-dimensional printing method, and the interval of printing regions is a region not to be printed, and therefore it is represented as pores in the final scaffold.

To investigate the liquid absorption of the prepared scaffold, water 0.05mL was loaded on the scaffold and the absorption of liquid according to the pore size was measured, and as a result, it was confirmed that the absorption of water was low when the pores of the lower layer of the scaffold was 300 *µ*m, but on the other hand, water was absorbed well in case of pores of 500 *µ*m or more. The lower support member could be manufactured by 3 layers in total, and the upper 1 layer was manufactured less than 300 *µ*m and the lower 2 and 3 layers were manufactured in the pore size of 500 *µ*m or more.

### Example 6: Manufacturing of artificial skin

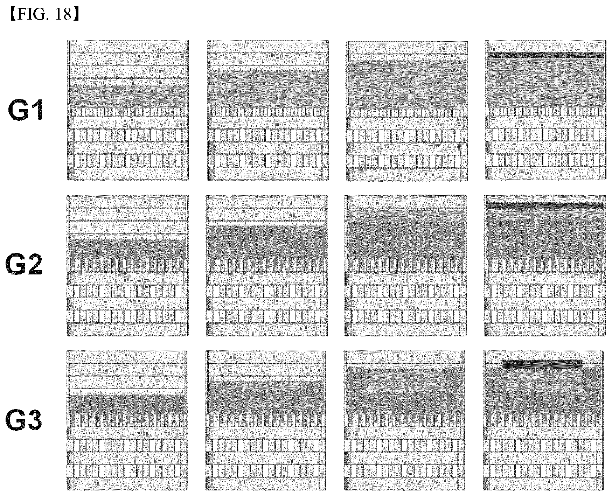

In the three-dimensional printing method of artificial skin, a contraction phenomenon was caused in the upper layer of the dermis layer in which a large amount of cells were aggregated, a separation phenomenon was occurred between the outer partitioning-wall member and the printed bioink (FIG. 17). To improve the separation phenomenon by a cell printing method, an experiment was progressed by setting three kinds of printing methods of Gl, G2 and G3. The schematic mimetic diagram of three kinds of printing methods of Gl, G2 and G3 were shown in FIG. 18.

Specifically, G1 printed so that cells were located in the entire regions of the dermis layer and epidermis layer, when printing the dermis layer and epidermis layer by the same method as the extrusion type of three-dimensional printing method of Example 3. G2 performed printing by the same method as the extrusion type of three-dimensional printing method of Example 3, but considering only cell interaction of the dermis layer and epidermis layer, bioink containing collagen without fibroblasts was printed in the lower layer of the dermis layer and collagen bioink containing fibroblasts was printed only in the upper layer, and the epidermis layer was printed so that cells were located in the entire regions. G3 performed printing by the same method as the extrusion type of three-dimensional printing method of Example 3, but cell non-containing collagen bioink was printed in the contact surface of the lower part and the outer partitioning-wall member of the dermis layer, and cell containing collagen bioink was printed only the inside spaced apart at an interval of less than 1000 micrometers with the outer partitioning-wall member, and the epidermis layer was printed so that cells were located on the formed dermis layer.

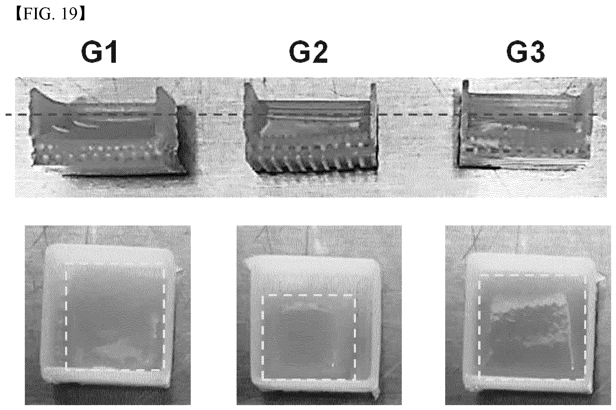

According to the same method as the experiment of measurement of degree of contraction of the cell layer of Experimental example 1, contraction of the cell layer of the prepared artificial skin (G1, G2, G3) was measured, and the photograph of the artificial skin at day 7 of the storage period was shown in FIG. 19.

As shown in the experimental result of FIG. 19, in G2 experiment compared to G1 experiment, the cell non-containing collagen bioink of the lower part of the dermis layer prevented contraction, and thus contraction in the direction of Z axis was improved, but the contraction phenomenon was shown as spaced apart from the outer partitioning-wall member by fibroblasts printed in the entire upper part of the dermis layer. G3 experiment confirmed that the contraction of the cell layer was prevented in the contact surface of both the lower part and outer partitioning-wall member of the dermis layer in which cell non-containing bioink was printed, and thus the total contraction was prevented. In other words, G2 experiment could prevent contraction of the cell layer in the lower part of the dermis layer, and G3 experiment is a method which can prevent all contraction of the cell layer in the contact surface of the lower part and outer partitioning-wall member of the dermis layer.

## Claims

1. A method for preparing a cell culture structure comprising an integrated culture scaffold and a cell layer, comprising steps of
preparing a culture scaffold comprising a porous lower support member which comprises two or more of print layers containing at least two or more of print lines located at an interval by an extrusion type of three-dimensional printing method, and pores which are formed by the print lines of different print layers located to have a certain crossing angle; and an outer partitioning-wall member formed along the upper outside of the lower support member, and
preparing a cell layer located on upper part of the culture scaffold.

2. The method for preparing a cell culture structure according to claim 1, wherein the method comprises filling a gelling polymer into the formed pores in the lower support member, and preparing a print line on the filling layer.

3. The method for preparing a cell culture structure according to claim 1, wherein the print lines of different print layers are crossed to be in a lattice pattern, and the width of the print lines is 0.1 to 0.5 mm, and the interval between adjacent print lines of the same print layer is 100 to 1000 um.

4. The method for preparing a cell culture structure according to claim 1, wherein the outer partitioning-wall member has a height of 1 to 5 times on the basis of the height of the cell layer of 1.

5. The method for preparing a dermis layer-like structure according to claim 1, wherein the thermoplastic resin is sprayed as a melt obtained by sealing it in a syringe and then heating it to 70 to 90 °C.

6. The method for preparing a cell culture structure according to claim 1, wherein the lower support member is prepared with one or more thermoplastic selected from the group consisting of polycaprolactone (PCL), poly(lactate-co-glycolate) (PLGA), poly lactic acid (PLA), polyurethane (PU), poly(lactide-co-caprolactone) (PLCL).

7. The method for preparing a cell culture structure according to claim 1, wherein in the lower support member, the interval of adjacent print lines of the same print layer is 100 to 1000 um.

8. The method for preparing a cell culture structure according to claim 7, wherein in the lower support member, the interval between adjacent print lines of the upper same print layer is 300 to 1,000 micrometers, and the interval between adjacent print lines of the lower same print layer is 100 to 500 micrometers.

9. The method for preparing a cell culture structure according to claim 1, wherein the cell layer is prepared by a three-dimensional printing method using bioink comprising a cell and a gelling polymer.

10. The method for preparing a cell culture structure according to claim 9, wherein the gelling polymer is one or more kinds selected from the group consisting of collagen, gelatin, Matrigel, alginate, agarose, decellularized tissue-derived cell ink, hyaluronic acid and fibrin gel.

11. The method for preparing a cell culture structure according to claim 1, wherein the cell layer is prepared as spaced at a distance of 50 micrometers to 5,000 micrometers from the outer partitioning-wall member.

12. The method for preparing a cell culture structure according to claim 1, wherein when the cell culture structure comprises two or more of different cell layers, the outer partitioning-wall member and the cell layers are alternately formed, or two or more of different cell layers are continuously prepared after preparing the outer partitioning-wall member.

13. The method for preparing a cell culture structure according to claim 1, comprising preparing a dermis layer by an extrusion type of three-dimensional printing method using bioink for preparing a dermis layer comprising a fibroblast and a gelling polymer, and cross-linking the gelling polymer.

14. The method for preparing a cell culture structure according to claim 1, wherein the height of the dermis layer is 0.1 to 3.5 mm.

15. The method for preparing a dermis layer-like structure according to claim 13, wherein the cross-linking is performed at the temperature of 37°C or more.

16. The method for preparing a cell culture structure according to claim 13, wherein the bioink for preparing a dermis layer contains collagen, fetal bovine serum (FBS) and human dermal fibroblasts.

17. The method for preparing a cell culture structure according to claim 13, wherein the bioink for preparing a dermis layer is that human dermal fibroblasts are mixed to a mixed solution of a collagen solution of 1%(w/v) to 10%(w/v) and fetal bovine serum in a volume ratio of 10:1 to 5:1, so that the density of the cell is 2.5 x 10⁵ cells/ml to 1 x 10⁶ cells/ml.

18. The method for preparing a cell culture structure according to claim 1, wherein the cell layers comprises a dermis layer and an epidermis layer and the method comprises printing bioink for preparing a epidermis layer comprising a keratinocyte and a gelling polymer, on upper part of the dermis layer by a three-dimensional printing method, and culturing the keratinocyte to proliferate and differentiate.

19. The method for preparing a cell culture structure according to claim 18, wherein the epidermis layer is printed by an extrusion type of three-dimensional printing method and is heated to a sol-gel phase transition temperature to solate the gelling polymer and to move the keratinocyte to the lower part of the epidermis layer.

20. The method for preparing a cell culture structure according to claim 18, wherein the gelling polymer is one or more kinds selected from the group consisting of collagen, gelatin, Matrigel, alginate, agarose, decellularized tissue-derived cell ink, hyaluronic acid and fibrin gel.

21. A culture scaffold comprising a porous lower support member which comprises two or more of print layers containing at least two or more of print lines located at an interval by an extrusion type of three-dimensional printing method, and pores which are formed by the print lines of different print layers located to have a certain crossing angle; and an outer partitioning-wall member formed along the upper outside of the lower support member.

22. The culture scaffold according to claim 21, wherein the print lines of different print layers are crossed to be in a lattice pattern, and the width of the print lines is 0.1 to 0.5 mm, and the interval between adjacent print lines of the same print layer is 100 to 1000 um.

23. The culture scaffold according to claim 21, wherein the outer partitioning-wall member has a height of 1 to 5 times on the basis of the height of the cell layer of 1.

24. The culture scaffold according to claim 21, wherein in the lower support member, the interval of adjacent print lines of the same print layer is 100 to 1000 um.

25. The culture scaffold according to claim 24, wherein in the lower support member, the interval between adjacent print lines of the upper same print layer is 300 to 1,000 micrometers, and the interval between adjacent print lines of the lower same print layer is 100 to 500 micrometers.

26. An integrated cell culture structure comprising a cell layer prepared by a three-dimensional printing method in the upper part of the lower support member and inside of the outer partitioning-wall member of the culture scaffold according to any one of claims 21 to 25.

27. The integrated cell culture structure according to claim 26, wherein the cell layer is a dermis layer comprising a fibroblast and a gelling polymer, and the height of the dermis layer is 0.1 to 3.5 mm.

28. The integrated cell culture structure according to claim 26, wherein the cell layer has a cross-sectional area reduction ratio of 25% based on a cross-sectional area of the initial cell layer of 100.
